# EUROPEAN PATENT SPECIFICATION

(11) **EP 2 351 775 B1**
(45) Date of publication and mention of the grant of the patent: **28.06.2017**
(21) Application number: 10193819.9
(22) Date of filing: 06.12.2010
(51) Int. Cl.: C07K 14/47, C12N 15/62

(54) **Fusion protein comprising ubiquitin or ubiquitin-like protein, membrane translocation sequence and biologically active molecule and use thereof**
Fusionsprotein mit Ubiquitin- oder ubiquitinähnlichem Protein, Membranverschiebungssequenz und biologisch aktives Molekül sowie Verwendung damit
Protéine de fusion comprenant de l'ubiquitine et protéine de type ubiquitine, séquence de translocation de membrane et molécule biologiquement active et utilisation associée

(30) Priority: 04.12.2009 KR 20090119912
(43) Date of publication of application: 03.08.2011
(73) Proprietor: Samsung Electronics Co., Ltd., Suwon-si, Gyeonggi-do, 443-742 (KR)
(72) Inventor: Lee, Jae-il, Gyeonggi-do (KR); Lee, Tae-soo, Gyeonggi-do (KR); Lee, Young-sun, Gyeonggi-do (KR)
(74) Representative: Grünecker Patent- und Rechtsanwälte PartG mbB

(56) References cited:
- WO-A1-2009/031836
- LOISON F ET AL: "A ubiquitin-based assay for the cytosolic uptake of protein transduction domains", MOLECULAR THERAPY, ACADEMIC PRESS, SAN DIEGO, CA, US, vol. 11, no. 2, 1 February 2005 (2005-02-01), pages 205-214, XP004723674, ISSN: 1525-0016, DOI: DOI:10.1016/J.YMTHE.2004.10.010
- VITTE ANNE-LAURE ET AL: "Intracellular delivery of peptides via association with ubiquitin or SUMO-1 coupled to protein transduction domains", BMC BIOTECHNOLOGY, BIOMED CENTRAL LTD. LONDON, GB, vol. 8, no. 1, 29 February 2008 (2008-02-29), page 24, XP021035694, ISSN: 1472-6750
- LIN Y ET AL: "INHIBITION OF NUCLEAR TRANSLOCATION OF TRANSCRIPTION FACTOR NF-KB BY A SYNTHETIC PEPTIDE CONTAINING A CELL MEMBRANE-PERMEABLE MOTIF AND NUCLEAR LOCALIZATION SEQUENCE", JOURNAL OF BIOLOGICAL CHEMISTRY, AMERICAN SOCIETY FOR BIOCHEMISTRY AND MOLECULAR BIOLOGY, INC, US, vol. 270, no. 24, 16 June 1995 (1995-06-16), pages 14255-14258, XP002050723, ISSN: 0021-9258, DOI: DOI:10.1074/JBC.270.24.14255
- EDENHOFER FRANK: "Protein Transduction Revisited: Novel Insights Into the Mechanism Underlying Intracellular Delivery of Proteins", CURRENT PHARMACEUTICAL DESIGN, vol. 14, no. 34, December 2008 (2008-12), pages 3628-3636, XP002633239, ISSN: 1381-6128
- JO D ET AL: "Epigenetic regulation of gene structure and function with a cell-permeable Cre recombinase", NATURE BIOTECHNOLOGY, NATURE PUBLISHING GROUP, NEW YORK, NY, US, vol. 19, no. 10, 1 October 2001 (2001-10-01), pages 929-933, XP002200323, ISSN: 1087-0156, DOI: DOI:10.1038/NBT1001-929
- ROISIN A ET AL: "Inhibition of HIV-1 replication by cell-penetrating peptides binding Rev", JOURNAL OF BIOLOGICAL CHEMISTRY, AMERICAN SOCIETY FOR BIOCHEMISTRY AND MOLECULAR BIOLOGY, INC, US, vol. 279, no. 10, 5 March 2004 (2004-03-05), pages 9208-9214, XP002277283, ISSN: 0021-9258, DOI: DOI:10.1074/JBC.M311594200

### SEQUENCE LISTING

<110> Samsung Electronics Co., Ltd
<120> Fusion Protein Comprising ubiquitin or Ubiquitin-like protein, Membrane Translocation sequence and Biologically Active Molecule and Use Thereof
<130> EP73712FZTRpau
<140> not yet assigned
   <141> 2009-12-04
<150> KR 10-2009-0119912
   <151> 2009-12-04
<160> 28
<170> KopatentIn 1.71
<210> 1
   <211> 16
   <212> PRT
   <213> Artificial sequence
<220>
   <223> membrane transfer domain
<400> 1
   Ala Ala Val Ala Leu Leu Pro Ala Val Leu Leu Ala Leu Leu Ala Pro
     1               5                  10                  15
<210> 2
   <211> 12
   <212> PRT
   <213> Artificial sequence
<220>
   <223> membrane transfer domain
<400> 2
   Ala Ala Val Leu Leu Pro Val Leu Leu Ala Ala Pro
     1               5                  10
<210> 3
   <211> 11
   <212> PRT
   <213> Artificial Sequence
<220>
   <223> membrane transfer domain
<400> 3
   Leu Leu His Leu Ser Leu Leu Ala Leu Ala Pro
     1               5                  10
<210> 4
   <211> 15
   <212> PRT
   <213> Artificial sequence
<220>
   <223> membrane transfer domain
<400> 4
   Leu Ala Val Ala Leu Leu Ala Ala Phe Leu Ile Ser Ala Ala Pro
   1 5 10 15
<210> 5
   <211> 13
   <212> PRT
   <213> Artificial sequence
<220>
   <223> membrane transfer domain
<400> 5
   Ala Leu Leu Ala Ala Leu Leu Ala Ala Pro Gly Ala Ala
   1 5 10
<210> 6
   <211> 8
   <212> PRT
   <213> Artificial Sequence
<220>
   <223> membrane transfer domain
<400> 6
   Leu Ile Ile Leu Leu Pro Val Val
   1 5
<210> 7
   <211> 16
   <212> PRT
   <213> Artificial sequence
<220>
   <223> membrane transfer domain
<400> 7
   Ala Ile Gly Leu Leu Trp Leu Leu Pro Leu Leu Leu Ser Thr Ala Ala
   1 5 10 15
<210> 8
   <211> 9
   <212> PRT
   <213> Artificial sequence
<220>
   <223> membrane transfer domain
<400> 8
   Leu Leu Leu Pro Ile Leu Ser Ser Ala
   1               5
<210> 9
   <211> 11
   <212> PRT
   <213> Artificial Sequence
<220>
   <223> membrane transfer domain
<400> 9
   Leu Leu Leu Leu Leu Leu Leu Pro Leu Leu Gly
   1               5                   10
<210> 10
   <211> 13
   <212> PRT
   <213> Artificial Sequence
<220>
   <223> membrane transfer domain
<400> 10
   Leu Gly Ala Ala Leu Leu Leu Leu Pro Leu Leu Ala Ala
   1               5                   10
<210> 11
   <211> 11
   <212> PRT
   <213> Artificial Sequence
<220>
   <223> membrane transfer domain
<400> 11
   Leu Leu Ser Val Leu Leu Leu Pro Leu Leu Gly
   1               5                   10
<210> 12
   <211> 11
   <212> PRT
   <213> Artificial Sequence
<220>
   <223> membrane transfer domain
<400> 12
   Leu Leu Leu Leu Pro Leu Leu Leu Leu Pro Ala
   1 5 10
<210> 13
   <211> 16
   <212> PRT
   <213> Artificial Sequence
<220>
   <223> membrane transfer domain
<400> 13
   Leu Leu Leu Ala Gly Leu Ala Ser Leu Leu Leu Pro Gly Ser Ala Ala
   1 5 10 15
<210> 14
   <211> 20
   <212> PRT
   <213> Artificial Sequence
<220>
   <223> membrane transfer domain
<400> 14
   Gly Ala Ala Ala Ala Pro Leu Leu Val Ala Val Ala Ala Leu Leu Leu
   1 5 10 15
   Gly Ala Ala Gly
   20
<210> 15
   <211> 9
   <212> PRT
   <213> Artificial Sequence
<220>
   <223> membrane transfer domain
<400> 15
   Gly Gly Gln Leu Pro Leu Leu Val Val
   1 5
<210> 16
   <211> 118
   <212> DNA
   <213> Artificial sequence
<220>
   <223> sense strand of polynucleotide encoding NLS-kFGF4-p53 polypeptide
<400> 16
   ttgaattcaa aaagaagaga aaggccgcgg tagcgctgct cccggcggtc ctgctggcct 60
   tgctggcgcc cgaaacattt tcagacctat ggaaactact tcctgaaaac aagcttaa 118
<210> 17
   <211> 118
   <212> DNA
   <213> Artificial sequence
<220>
   <223> antisense strand of polynucleotide encoding NLS-kFGF4-p53 polypeptide
<400> 17
   ttaagcttgt tttcaggaag tagtttccat aggtctgaaa atgtttcggg cgccagcaag 60
   gccagcagga ccgccgggag cagcgctacc gcggcctttc tcttcttttt gaattcaa 118
<210> 18
   <211> 38
   <212> DNA
   <213> Artificial Sequence
<220>
   <223> forward primer for synthesis of polynucleotide encoding human ubiquitin
<400> 18
   ttggatccgg tagtggaagc atgcagattt tcgtgaaa 38
<210> 19
   <211> 28
   <212> DNA
   <213> Artificial Sequence
<220>
   <223> reverse primer for synthesis of polynucleotide encoding human ubiquitin
<400> 19
   ttgaattcac cacgaagtct caacacaa 28
<210> 20
   <211> 36
   <212> DNA
   <213> Artificial Sequence
<220>
   <223> forward primer for synthesis of polynucleotide encoding human p18
<400> 20
   ttaagcttat ggccgagcct tgggggaacg agttgg 36
<210> 21
   <211> 30
   <212> DNA
   <213> Artificial Sequence
<220>
   <223> reverse primer for synthesis of polynucleotide encoding human p18
<400> 21
   ttctcgagtt attgaagatt ttgtggctcc 30
<210> 22
   <211> 24
   <212> DNA
   <213> Artificial Sequence
<220>
   <223> forward primer for synthesis of polynucleotide encoding human p18 site-directed mutant
<400> 22
   cttagaggtg ctaatcccga tttg 24
<210> 23
   <211> 24
   <212> DNA
   <213> Artificial Sequence
<220>
   <223> reverse primer for synthesis of polynucleotide encoding human p18 site-directed mutant
<400> 23
   gtctttcaaa tcgggattag cacc 24
<210> 24
   <211> 849
   <212> DNA
   <213> Artificial Sequence
<220>
   <223> polynucleotide encoding human ubiquitin-NLS-MTD-p53-p18 polypeptide
<400> 24
   atgcagattt tcgtgaaaac ccttacgggg aagaccatca ccctcgaggt tgaaccctcg      60
   gatacgatag aaaatgtaaa ggccaagatc caggataagg aaggaatacc tcctgatcag     120
   cagagactga tctttgctgg caagcagctg gaagatggac gtactttgtc tgactacaat     180
   attcaaaagg agtctactct tcatcttgtg ttgagacttc gtggtggtga attcaaaaag     240
   aagagaaagg ccgcggtagc gctgctcccg gcggtcctgc tggccttgct ggcgcccgaa     300
   acattttcag acctatggaa actacttcct gaaaacaagc ttatggccga gccttggggg     360
   aacgagttgg cgtccgcagc tgccaggggg gacctagagc aacttactag tttgttgcaa     420
   aataatgtaa acgtcaatgc acaaaatgga tttggaagga ctgcgctgca ggttatgaaa     480
   cttggaaatc ccgagattgc caggagactg ctacttagag gtgctaatcc cgatttgaaa     540
   gaccgaactg gtaatgctgt cattcatgat gcggccagag caggtttcct ggacacttta     600
   cagactttgc tggagtttca agctgatgtt aacatcgagg ataatgaagg gaacctgccc     660
   ttgcacttgg ctgccaaaga aggccacctc cgggtggtgg agttcctggt gaagcacacg     720
   gccagcaatg tggggcatcg gaaccataag ggggacaccg cctgtgattt ggccaggctc     780
   tatgggagga atgaggttgt tagcctgatg caggcaaacg gggctggggg agccacaaat     840
   cttcaataa                                                             849
<210> 25
   <211> 282
   <212> PRT
   <213> Artificial Sequence
<220>
   <223> human ubiquitin-NLS-MTD-p53-p18 polypeptide
<400> 25
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Arg Leu Ile Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu His Leu Val Leu Arg Leu Arg Gly Gly Glu Phe Lys Lys
   65                  70                  75                  80
   Lys Arg Lys Ala Ala Val Ala Leu Leu Pro Ala Val Leu Leu Ala Leu
                   85                  90                  95
   Leu Ala Pro Glu Thr Phe Ser Asp Leu Trp Lys Leu Leu Pro Glu Asn
               100                 105                 110
   Lys Leu Met Ala Glu Pro Trp Gly Asn Glu Leu Ala Ser Ala Ala Ala
           115                 120                 125
   Arg Gly Asp Leu Glu Gln Leu Thr Ser Leu Leu Gln Asn Asn Val Asn
       130                 135                 140
   Val Asn Ala Gln Asn Gly Phe Gly Arg Thr Ala Leu Gln Val Met Lys
   145                 150                 155                 160
   Leu Gly Asn Pro Glu Ile Ala Arg Arg Leu Leu Leu Arg Gly Ala Asn
                   165                 170                 175
   Pro Asp Leu Lys Asp Arg Thr Gly Asn Ala Val Ile His Asp Ala Ala
               180                 185                 190
   Arg Ala Gly Phe Leu Asp Thr Leu Gln Thr Leu Leu Glu Phe Gln Ala
           195                 200                 205
   Asp Val Asn Ile Glu Asp Asn Glu Gly Asn Leu Pro Leu His Leu Ala
       210                 215                 220
   Ala Lys Glu Gly His Leu Arg Val Val Glu Phe Leu Val Lys His Thr
   225                 230                 235                 240
   Ala Ser Asn Val Gly His Arg Asn His Lys Gly Asp Thr Ala Cys Asp
                   245                 250                 255
   Leu Ala Arg Leu Tyr Gly Arg Asn Glu Val Val Ser Leu Met Gln Ala
               260                 265                 270
   Asn Gly Ala Gly Gly Ala Thr Asn Leu Gln
           275                 280
<210> 26
   <211> 5
   <212> PRT
   <213> Artificial sequence
<220>
   <223> nucleus localization signal domain
<400> 26
   Lys Lys Lys Arg Lys
   1               5
<210> 27
   <211> 7
   <212> PRT
   <213> Artificial Sequence
<220>
   <223> nucleus localization signal domain
<400> 27
   Pro Lys Lys Lys Arg Lys Val
   1               5
<210> 28
   <211> 16
   <212> PRT
   <213> Artificial Sequence
<220>
   <223> nucleous localization signal domain
<400> 28
   Lys Arg Pro Ala Ala Thr Lys Lys Ala Gly Gln Ala Lys Lys Lys Lys
   1               5                   10                  15

## Description

### BACKGROUND

### 1. Field

The present disclosure relates to a transmembrane fusion protein including ubiquitin or ubiquitin-like protein, a membrane translocation sequence that is linked to the C-terminus of the ubiquitin or ubiquitin-like protein, and a biologically active molecule that is linked to the C-terminus of the membrane translocation sequence, a polynucleotide encoding the transmembrane fusion protein, a recombinant expression vector including the polynucleotide sequence, a cell transformed by the recombinant expression vector, and a method of delivering the biologically active molecule into a cell using the transmembrane fusion protein.

### 2. Description of the Related Art

Since it was reported that various diseases result from abnormal activities of a protein in cells, attempts have been made to develop drugs for treating diseases by regulating the abnormal activity of a protein. In particular, research into peptide, polypeptide and protein drugs has been carried out based on enzyme-protein or protein-protein interactions which may specifically regulate biological activity. Even though peptides, polypeptides, and proteins have superior biological selectivities and effects, it is difficult for them to penetrate a cell membrane due to their size and various biochemical properties. Since peptides, polypeptides, and proteins cannot be directly delivered into cells, their practical use as effective drugs has had limitations.

A variety of attempts have been made to deliver peptides, polypeptides, or proteins into cells. Recently, transmembrane peptides have been used to deliver peptides, polypeptides, or proteins into cells. Research into the cell-penetrating mechanisms of proteins has been carried out since it was reported in 1988 that Tat protein of HIV-1 was actively transported into a cell when used with a culture medium. Since then, diverse research has been conducted into various peptides that penetrate a cell membrane, for example, signal sequences (transmembrane sequence) or the cytoplasmic penetration sequences existing in Antp protein of a drosophila, VP22 protein of herpes simplex virus (HSV), and fibroblast growth factor (FGF).
Loison, F. et al., "A ubiquitin-based assay for the cytosolic uptake of protein transduction domains", MOLECULAR THERAPY, ACADEMIC PRESS, SAN DIEGO, CA, US, vol. 11, no. 2, 1 February 2005, pages 205-214, discloses fusion proteins comprising from the N- to the C-terminus: (i) a PTD (protein transduction domain), e.g. melittin or the TAT peptide from HIV, (ii) a ubiquitin moiety, and (iii) a cargo peptide, such as PEP, HSP70 and BCR-XL. The authors show that deubiquitinating enzyme (DUB) activity can be used to determine cytosol localisation of cargo proteins by visualization of DUB cleavage products.
Similarly, Vitte, Anne-Laure et al., "Intracellular delivery of peptides via association with ubiquitin or SUMO-1 coupled to protein transduction domains" BMC BIOTECHNOLOGY, BIOMED CENTRAL LTD. LONDON, GB, vol. 8, no. 1, 29 February 2008, page 24, discloses fusion proteins comprising from the N- to the C-terminus: (i) a FLAG epitope, (ii) a cleavage site for protease, (iii) a PTD, (iv) a human ubiquitin or SUMO-I, and (v) either GFP or the HA epitope.

According to recent research, a membrane translocation sequence (MTS) directly penetrates a cell membrane without using a receptor/transporter, while other cell penetrating peptides (CPPs) need to be delivered using a receptor/transporter. Thus, an MTS is not dependent on the expression of a receptor/transporter. In addition, since an MTS is transferred independently of endocytosis, an MTS is more efficiently transferred into a cell. An MTS can migrate cell-to-cell, although other CPPs cannot migrate in the same way. Due to theses features, membrane translocation sequences are attracting a lot of attention.

However, due to its hydrophobicity, it is difficult to apply an MTS in animal tests. For example, if a fusion protein including an MTS is expressed in a recombinant cell, the fusion protein including the MTS is obtained as an inclusion body in a heterogenous form which has poor stability. Further, it is difficult to obtain active fusion protein from the inclusion body.

Thus, there is still a need to develop a fusion protein including an MTS that is stably transferred into cells.

### SUMMARY

Provided are a transmembrane fusion protein according to claim 1 including ubiquitin or a ubiquitin-like protein, a membrane translocation sequence that is linked to the C-terminus of the ubiquitin or ubiquitin-like protein, and a biologically active molecule that is linked to the C-terminus of the membrane translocation sequence, a polynucleotide encoding the transmembrane fusion protein, a recombinant expression vector including the polynucleotide sequence, a cell transformed by the recombinant expression vector, and
an *in vitro* method of delivering the transmembrane fusion protein to a cell.

In an embodiment, the method of delivering the transmembrane fusion protein includes contacting the transmembrane fusion protein with the cell.

A method of producing the transmembrane fusion protein is also disclosed. In an embodiment, the method includes culturing a host cell transformed by a polynucleotide encoding the transmembrane fusion protein under conditions which allow expression of the transmembrane fusion protein; and recovering the transmembrane fusion protein from the host cell culture.

Additional aspects will be set forth in part in the description which follows and, in part, will be apparent from the description, or may be learned by practice of the presented embodiments.

### BRIEF DESCRIPTION OF THE DRAWINGS

These and/or other aspects will become apparent and more readily appreciated from the following description of the embodiments, taken in conjunction with the accompanying drawings of which:
FIG 1 is a schematic diagram illustrating transfer of a fusion protein according to an embodiment of the present invention into a cell and/or a nucleus;
FIG. 2A to FiG. 2C show fluorescent microscopic images visualizing the *in* vivo distribution of a fusion protein according to an embodiment of the present invention or controls in mouse cells isolated after intraperitoneal injection of the fusion protein or controls into the mice; and
FIG. 3 is a graph showing tumor volume as a function of injection time of p53-p18 contained in a fusion protein according to an embodiment of the present invention or a control fusion protein.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

According to an embodiment of the present invention, a transmembrane fusion protein of claim 1 includes: ubiquitin or a ubiquitin-like protein; a membrane translocation sequence that is linked to the C-terminus of the ubiquitin or ubiquitin-like protein; and a biologically active molecule that is linked to the C-terminus of the membrane translocation sequence.

The term "transmembrane fusion protein "used herein indicates a fusion protein having the capability of delivering a biologically active molecule into a cell or a nucleus in vitro and/or in vivo. In addition, a "membrane translocation sequence (MTS)" is a polypeptide having an amino acid sequence which may penetrate a phospholipid bilayer of a cell membrane. An MTS has a single hydrophobic region at the N-terminus and is formed of relatively few amino acids (7 to 17 amino acids) in a flexible helix structure. Thus, an MTS has hydrophobicity.

The term, "link/linked", as used herein, shall mean the linking of two or more biomolecules so that the biological functions, activities, and/or structure associated with the biomolecules are at least retained. In reference to polypeptides, the term means that the linking of two or more polypeptides results in a fusion polypeptide that retains at least some of the respective individual activities of each polypeptide component. The two or more polypeptides may be linked directly or via a linker.

Ubiquitin (Ub) is a highly conserved protein found in all eukaryotic cells that includes 76 amino acids. Ubiquitin exhibits perfect homology among species as diversed as insects, trout, and humans. In addition, ubiquitin is stable against pH changes, not easily denatured at high temperature, and stable against proteases. Thus, if ubiquitin is fused to an MTS, the water-insolubility of the MTS may be reduced.

A ubiquitin-like protein (UbL) is a protein having properties that are similar to those of ubiquitin. Examples of a ubiquitin-like protein include Nedd8, SUMO-1, SUMO-2, NUB1, PIC1, UBL3, UBL5, and ISG15

The ubiquitin or ubiquitin-like protein in the transmembrane fusion protein may be selected from the group consisting of wild-type ubiquitin, a wild-type ubiquitin-like protein, mutant ubiquitin and a mutant ubiquitin-like protein. A mutant ubiquitin is obtained by changing the amino acid sequence of wild-type ubiquitin into another amino acid sequence. For example, a mutant ubiquitin may be prepared by replacing a Lys of wild-type ubiquitin with an Arg or by replacing the RGG residues of the C-terminus of wild-type ubiquitin with RGA residues. In mutant ubiquitins prepared by replacing Lys of wild-type ubiquitin with Arg, Lys residues that exist at the 6^{th}, 11^{th}, 27^{th}, 29^{th}, 33^{rd}, 48^{th}, and 63^{rd} amino acid positions may be replaced with Arg independently or in any combination.

The ubiquitin or ubiquitin-like protein in the transmembrane fusion protein has an amino acid sequence at the C-terminus which can or cannot be cleaved by a protease. An amino acid sequence that is cleaved by a protease may be identified by searching a database that is known in the art. For example, the tool PeptideCutter, available on the internet from the ExPASy (Expert Protein Analysis System) proteomics server of the Swiss Institute of Bioinformatics may be used to identify a protease and the amino acid sequence cleaved by the protease.

In some embodiments, if the amino acid sequence recognized by a protease is contained in ubiquitin or the ubiquitin-like protein, the ubiquitin or ubiquitin-like protein in the transmembrane fusion protein may be cleaved by the protease existing in the cell after the fusion protein is transferred into the cell, so that the Ub or UbL is released from the fusion protein and the biologically active molecule may perform desirable functions. Even though the biologically active molecule in the transmembrane fusion protein still includes the MTS after the ubiquitin or ubiquitin-like protein is removed by proteolytic cleavage, the MTS does not affect the function of the biologically active molecule since the polypeptide sequence of the MTS is short. In some embodiments, even when the ubiquitin or ubiquitin-like protein is not cleaved from the transmembrane fusion protein by a cellular protease, the biologically active molecule may perform desirable functions. Likewise, the ubiquitin or ubiquitin-like protein may be cleaved prior to enteming the cell, e.g. ubiquitin C-terminal hydrolases in the blood of a subject to which the transmembrane fusion protein was administered, and the cleavage product that contains the MTS and the biologically active molecule will be delivered inside the cells.

The MTS in the transmembrane fusion protein may be any polypeptide having an amino acid sequence that may pass through a phospholipid bilayer of a cell membrane without limitation. For example, the MTS may be a polypeptide having a sequence selected from SEQ ID NOS: 1 to 15.

The "biologically active molecule" in the transmembrane fusion protein is a polypeptide or protein with a desired biological activity to target into a cell that may be conjugated to an MTS and transferred into a cell.

The biologically active molecule in the transmembrane fusion protein is a peptide or protein having a length of at least two amino acids. The polypeptide may be a protein related to cell immortality such as SV40 large T antigen and telomerase; an anti-apoptotic protein such as mutant p53 and BclxL; an antibody; a cancer gene product such as ras, myc, human papilloma virus E6/E7, and adenovirus Ela; a cell cycle regulating protein such as cyclin and cyclin-dependent kinase; a green fluorescent protein; or an enzyme such as β-galactosidase and chloramphenicol acetyl transferase, but is not limited thereto.

The biologically active molecule contained in the transmembrane fusion protein may be a hydrophilic protein, but is not limited thereto. In addition, the biologically active molecule may be a hormone, a hormone-like protein, an enzyme, an enzyme inhibitor, a signal transduction protein or parts thereof, an antibody or parts thereof, a single chain antibody, a binding protein or a binding domain thereof, an antigen, an adhesion protein, a structural protein, a regulatory protein, toxoprotein, cytokine, transcription factor, blood clotting factor, a vaccine, or the like, but is not limited thereto.

For example, the biologically active molecule contained in the transmembrane fusion protein may be p53, p18, p53-p18, p21, p25, p57, p16, p15, NIP71, neuroregulin 1, PTEN tumor suppressor, ARF tumor suppressor, APC, CD95, folliculin, MEN1, BRCA1, Von Hippel-Lindau tumor suppressor, RKIP, nm23, endostatin, insulin, insulin-like growth factor 1 (IGF-1), growth factor, erythropoietin, granulocyte-colony stimulating factor (G-CSF), granulocyte/macrophage-colony stimulating factor (GM-CSF), interferon-α, interferon-β, interferon-γ, interleukin-1 α and β, interleukin-3, interleukin-4, interleukin-6, interleukin-2, epidermal growth factor (EGF), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRH-II), gonadorelin, goserelin, histrelin, leuprorelin, lypressin, octreotide, oxytocin, pitressin, secretin, sincalide, terlipressin, thymopentin, thymosine α1, triptorelin, bivalirudin, carbetocin, cyclo-sporine, exedine, lanreotide, luteinizing hormone-releasing hormone (LHRH), nafarelin, parathyroid hormone, pramlintide, enfuvirtide (T-20), thymalfasin, or ziconotide, but is not limited thereto.

The transmembrane fusion protein may further include a nuclear localization signal domain when the biologically active molecule needs to be delivered into the nucleus.

A "nuclear localization signal domain (NLS) " is an amino acid sequence capable of passing through the nuclear membrane of a cell which is contained in proteins transported from cytoplasm to nucleus. Here, the amino acid sequence is not limited as long as the sequence possesses NLS function. The polypeptide that may be used as the NLS may be KKKRK (SEQ ID NO: 26), PKKKRKV(SEQ ID NO: 27), KRPAATKKAGQAKKKK (SEQ ID NO: 28), or the like, but is not limited thereto.

The NLS may be located in at least one region of the transmembrane fusion protein selected from the group consisting of the C-terminus of ubiquitin or the ubiquitin-like protein, the C-terminus of the MTS, and the C-terminus of the biologically active molecule.

"Isolated," when used to describe the various polypeptides or polynucleotides disclosed herein, means a polypeptide or polynucleotide that has been identified and separated and/or recovered from a component of its natural environment. The term also embraces recombinant polynucleotides and polypeptides and chemically synthesized polynucleotides and polypeptides.

According to an embodiment of the present invention, there is provided a composition for delivering the biologically active molecule to a cell. The composition includes the transmembrane fusion protein disclosed herein; and a pharmaceutically acceptable carrier. The transmembrane fusion protein may be present in the composition in an amount such that the biologically active molecule is delivered in a therapeutically effective amount.

According to an embodiment of the present invention, a polynucleotide is provided which encodes a transmembrane fusion protein that includes: ubiquitin or a ubiquitin-like protein; an MTS that is linked to the C-terminus of the ubiquitin or ubiquitin-like protein; and a biologically active molecule that is linked to the C-terminus of the MTS.

A "polynucleotide" as used herein is a polymer of deoxyribonucleotides or ribonucleotides. The polynucleotide can be a single-stranded or double-stranded nucleic acid. The polynucleotide includes a RNAgenome sequence, a cDNAsequence, a RNA sequence transcribed from the cDNA, and an analog of a natural polynucleotide, unless otherwise indicated herein.

The polynucleotide includes not only the nucleotide sequence encoding the amino acid sequence of the transmembrane fusion protein, but also its complementary sequence. The complementary sequence may be a perfectly complementary sequence or a substantially complementary sequence to the nucleotide sequence. That is, the complementary sequence may be a sequence that is hybridized with, for example, a nucleotide sequence that encodes an amino acid sequence of the transmembrane fusion protein under stringent conditions known in the art. Specifically, stringent conditions mean, for example, hybridization to DNA in 6×SSC at about 45°C, followed by one or more washes in 0.2×SSC/0.1% SDS at about 50°C-65°C.

A nucleotide sequence encoding a ubiquitin or a ubiquitin-like protein contained in the transmembrane fusion protein may be derived from a mammal, for example, a human. In addition, the nucleotide sequence encoding a ubiquitin or a ubiquitin-like protein may be obtained by searching a known nucleotide sequence database, for example, the National Center for Biotechnology Information (NCBI) Entrez databases or the European Molecular Biology Laboratory-European Bioinformatics Institute (EMBL-EBI) databases.

According to another embodiment of the present invention, a recombinant vector includes: a polynucleotide encoding a transmembrane fusion protein including: ubiquitin or a ubiquitin-like protein; an MTS that is linked to the C-terminus of the ubiquitin or ubiquitin-like protein; and a biologically active molecule that is linked to the C-terminus of the MTS; and a promoter operatively linked to the polynucleotide sequence.

The term "operatively linked" used herein indicates a functional binding of a nucleotide expression controlling sequence (e.g., promoter sequence) to another nucleotide sequence, and the nucleotide expression controlling sequence may control transcription and/or translation of the other nucleotide sequence thereby.

The recombinant vector may be an expression vector that stably expresses the transmembrane fusion protein in a host cell. The expression vector may be a vector known in the art that is used to express an exogenous protein in plants, animals, or microorganisms. The recombinant vector may be formed using various methods known in the art.

The recombinant vector may be constructed for use in a prokaryotic or a eukaryotic host cell. For example, if the vector is an expression vector for use in a prokaryotic host cell, the vector may include a promoter capable of initiating transcription, such as p_{L}^{λ} promoter, *trp* promoter, *lac* promoter, *tac* promoter, and T7 promoter; a ribosome-binding site to initiate translation; and a transcription/translation termination sequence. When a eukaryotic cell is used as the host cell, the vector may contain an origin of replication operating a eukaryotic cell, e.g., a f1 replication origin, a SV40 replication origin, a pMB1 replication origin, an adeno replication origin, an AAV replication origin, and a BBV replication origin, but is not limited thereto. The promoter used in an expression vector for a eukaryotic host cell may be a promoter derived from a mammalian cell genome (for example, a metallothionein promoter) or a promoter derived from a mammalian virus (for examplean adenovirus anaphase promoter, vaccunia virus 7.5K promoter, a SV40 promoter, a cytomegalo virus promoter, or *tk* promoter of HSV). The expression vector for a eukaryotic host cell may in general also include a polyadenylated sequence as a transcription termination sequence.

According to an embodiment of the present invention, a host cell is provided which includes the polynucleotide sequence encoding the transmembrane fusion protein disclosed herein. The host cell may be a cell that is transformed by the recombinant vector including the polynucleotide encoding the transmembrane fusion protein.

The host cell may include the polynucleotide encoding the transmembrane fusion protein including: ubiquitin or ubiquitin-like protein; an MTS that is linked to the C-terminus of the ubiquitin or ubiquitin-like protein; and a biologically active molecule that is linked to the C-terminus of the MTS in a genome, or may include the recombinant vector including the polynucleotide sequence.

The host cell that can stably and continuously clone or express the recombinant vector may be any host cell that is known in the art. Examples of a prokaryotic host cell are *E. coli* JM109, *E. coli* BL21, *E*. *coli* RR1, *E. coli* LE392, *E*. *coli* B, *E. coli* X 1776, *E*. *coli* W3110, strains of Bacillus species such as Bacillus subtillis or Bacillus thuringiensis, intestinal bacteria and strains such as salmonella typhymurum, Serratia marcescens, or various Pseudomonas species. Examples of a eukaryotic host cell are *Saccharomyces cerevisiae,* an insect cell, a plant cell, or an animal cell such as Chinese hamster ovary (CHO), W138, BHK, COS-7, 293, HepG2, 3T3, RIN, and MDCK cell lines.

The polynucleotide or the recombinant vector including the polynucleotide may be delivered to a host cell using a delivery method known in the art. The delivery method may vary according to the host cell. If the host cell is a prokaryotic cell, a CaCl₂ method or an electroporation method may be used. If the host cell is a eukaryotic cell, microinjection, calcium phosphate precipitation, electroporation, liposome-mediated transfection, or gene bombardment may be used. However, the delivery method is not limited thereto.

The transformed host cell may be selected using a phenotype expressed by a selectable marker and a method known in the art. For example, if the selectable marker is a specific antibiotic resistance gene, the transformed host cell may be distinguished by culturing the transformed cell in a medium containing the antibiotic.

According to another embodiment of the present invention, a method of preparing a transmembrane fusion protein is disclosed. The method comprises: culturing a cell including a polynucleotide sequence encoding a transmembrane fusion protein including: ubiquitin or ubiquitin-like protein; an MTS that is linked to the C-terminus of the ubiquitin or ubiquitin-like protein; and a biologically active molecule that is linked to the C-terminus of the MTS under conditions at which the transmembrane fusion protein is expressed; and isolating transmembrane fusion protein expressed in a culture medium. The host cell may be a bacterial host cell.

The cultivation of the transformed cell may be performed using a method known in the art. For example, the transformed cell can be inoculated into a YT liquid culture medium and cultured. When cell density reaches a predetermined level, isopropyl-beta-thio galactopyranoside (IPTG) can be added to the culture medium to induce the expression of the protein by the *lac*Z promoter. Subsequently, protein expressed within the cell or secreted to the culture medium may be collected.

The protein expressed within the cell or secreted to the culture medium may be isolated using a purification method that is known in the art. For example, solubility fractionation using ammonium sulfate, size classification and filtration, and various chromatography separation methods (separation according to size, charge, hydrophobicity, or hydrophilicity) may be used to obtain isolated proteins. For example, if the transmembrane fusion protein includes glutathione S-transferase (GST), the protein may be efficiently isolated using a glutathione resin column. If the transmembrane fusion protein includes 6x His sequence, a Ni²⁺-NTA His-binding resin column may be used to obtain the desired protein.

According to another embodiment of the present invention, an in vitro method of delivering a transmembrane fusion protein into a cell is provided.

The method may include contacting the transmembrane fusion protein with the cell.

In a further aspect, the cell contacted may be an individual cell, a cell in a tissue, a cell in an organ, or a cell in an individual. Contacting the transmembrane fusion protein with a cell can be performed directly in vitro by exposing the cell to the transmembrane fusion protein dissolved in a buffer solution. Contacting the transmembrane fusion protein with the cell in vivo may include administering the transmembrane fusion protein to a subject.

The subject may be a mammal, for example a human. Administration of the transmembrane fusion protein may be parenteral administration to an individual of the transmembrane fusion protein. The transmembrane fusion protein may be alone or in a composition with a pharmaceutically acceptable excipient. Parenteral administration may be performed using intravenous injection, subcutaneous injection, intramuscular injection, intraperitoneal injection, endodermal injection, topical administration, intranasal administration, lung administration, rectal administration, or the like. Since the administered transmembrane fusion protein includes an MTS, the biologically active molecule may be delivered into a cell contacted by the transmembrane fusion protein, which has been administered using a method described above.

The present invention will be described in further detail with reference to the following examples. These examples are for illustrative purposes only and are not intended to limit the scope of the invention.

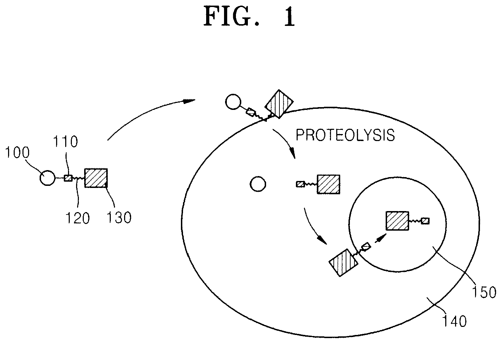

FIG. 1 schematically illustrates transfer of a fusion protein according to an embodiment of the present invention into a cell and/or a nucleus.

In a schematic diagram, if the amino acid sequence recognized by a protease is contained in ubiquitin or the ubiquitin-like protein 100, the ubiquitin or ubiquitin-like protein 100 in the transmembrane fusion protein may be cleaved by the protease existing in the cell 140 after the fusion protein is transferred into the cell 140, so that the ubiquitin or the ubiquitin-like protein 100 is released from the fusion protein and the biologically active molecule 130 may perform desirable functions in cytosol. Alternatively, the biologically active molecule 130 may perform desirable functions in nucleus 150 if the fusion protein has NLS 110. Even though the biologically active molecule 130 in the transmembrane fusion protein still includes the MTS 120 after the ubiquitin or ubiquitin-like protein 100 is removed by proteolytic cleavage, the MTS 120 does not affect the function of the biologically active molecule 130 since the polypeptide sequence of the MTS 120 is short.

### Example 1 : Preparation of expression vector for a transmembrane fusion protein including ubiquitin, NLS, MTS, and p53-p18

An expression vector was prepared for producing a transmembrane fusion protein including the hydrophilic polypeptide ubiquitin, a NLS, an MTS, and the biologically active polypeptide p53-p18 which are sequentially linked.

In order to produce the transmembrane fusion protein, a pET-NLS-kFGF4-p53 expression vector was prepared that included a polynucleotide sequence encoding the NLS polypeptide sequence KKKRK ( SEQ ID NO: 26), a polynucleotide sequence encoding a known MTS sequence, the leader sequence of kFGF4 growth factor (AAVALLPAVLLALLAP; SEQ ID NO: 1), and a polynucleotide sequence encoding the N-terminal fragment of p53 polypeptide (mdm2 binding domain). The expression vector was prepared by annealing the sense and antisense strands of oligonucleotide encoding NLS-kFGF4-p53 polypeptide (sense strand:
5'ttgaattcaaaaagaagagaaaggccgcggtagcgctgctcccggcggtcctgctggccttgctggcgcccgaaacat tttcagacctatggaaactacttcctgaaaacaagcttaa3'; SEQ ID NO: 16, antisense strand: 5' ttaagcttgttttcaggaagtagtttccataggtctgaaaatgtttcgggcgccagcaaggccagcaggaccgccgggagc agcgctaccgcggcctttctcttctttttgaattcaa3'; SEQ ID NO: 17), and then cleaving the annealed product with the restriction enzymes *Eco*RI and *Hind*III*.* The cleaved fragments were inserted into the vector pET22b that was cleaved using the same restriction enzymes.

Then, polymerase chain reaction (PCR) was used to synthesize polynucleotides of the known cDNA sequence of human ubiquitin and the known cDNA sequence of human p18 (CDKN2C). In the PCR synthesis of the human ubiquitin polynucleotide fragment, 5'ttggatccggtagtggaagcatgcagattttcgtgaaa3' (SEQ ID NO: 18) and 5'ttgaattcaccacgaagtctcaacacaa3' (SEQ ID NO: 19) were used as the forward and the reverse primer, respectively. The forward and reverse primers each included restriction sites for *B*amHI and *E*coRI. In the PCR synthesis of the human p18 polynucleotide fragment, 5' ttaagcttatggccgagccttgggggaacgagttgg3' (SEQ ID NO: 20) was used as the forward primer, and 5' ttctcgagttattgaagattttgtggctcc3' (SEQ ID NO: 21) was used as the reverse primer. The forward and reverse primers each included restriction sites for *Hind*III and *Xho*I*.* Then, to improve the hydrophilicity of the p18, the codon for the 71^{st} amino acid, phenylalanine (F), was replaced with the codon for asparagine (N) using 5'cttagaggtgctaatcccgatttg3' (SEQ ID NO: 22) as the forward primer and 5'gtctttcaaatcgggattagcacc3'(SEQ ID NO: 23) as the reverse primer to perform site-directed mutagenesis of the nucleic acid sequence. The mutant was named "p18(F71 N)".

PCR was performed by a known method in the art using a GeneAmp PCR System 9700 (Applied Biosystem). The PCR products were purified using a QIAquick Multiwell PCR Purification kit (Qiagen) and each was cloned into a pGEM-T Easy vector (Promega). Then, for each recombinant pGEM-T Easy vector created, a competent cell (*E. Coli* DH5α) was transformed with the vector, and the plasmid was subsequently isolated from a culture of the transformed cell using QIAprep Spin Miniprep kit (Qiagen).

The pGEM-T Easy vector including the cDNA of human ubiquitin was cleaved using *Bam*HI and *Eco*RI, and the pGEM-T Easy vector including the cDNA of human p18(F71 N) was cleaved using *Hind*III and *Xho*I*.* Then, both fragments were inserted into the pET-NLS-kFGF4-p53 expression vector. This final vector prepared, capable of expressing the human ubiquitin-NLS-MTS-p53-p18(F71 N) fusion protein, was named pET-Ub-NLS-kFGF4-p53-p18(F71 N). The polypeptide sequence of the human ubiquitin-NLS-MTS-p53-p18 fusion protein inserted into the pET vector and polynucleotide sequence encoding the polypeptide sequence are shown as SEQ ID NOS: 25 and 24, respectively.

In the polynucleotide sequence of SEQ ID NO: 24, the 1^{st} to 228^{th} nucleotides encode human ubiquitin, the 235^{th} to 249^{th} nucleotides encode the NLS, the 250^{th} to 297^{th} nucleotides encode the KFG4 leader, the 298^{th} to 336^{th} nucleotides encode p53, and the 343^{rd} to 849^{th} nucleotides encode p18. The locations of each polypeptide in the amino acid sequence, as shown in SEQ ID NO: 25, may be inferred from the corresponding locations in the nucleotide sequence of SEQ ID NO: 24.

A control transmembrane fusion protein expression vector, analogous to pET-Ub-NLS-kFGF4-p53-p18(F71 N) vector, but lacking ubiquitin at the NH2-terminus of the fusion protein, was also prepared as described above. The control fusion protein vector is denoted as pET-NLS-kFGF4-p53-p18(F71N) vector and the control fusion protein is denoted as NLS-kFGF4-p53-p18(F71N).

### Example 2 : Expression and purification of the transmembrane fusion protein

The transmembrane fusion protein was over-expressed in *E. coli* BL21(DE3) transformed with the pET-Ub-NLS-kFGF4-p53-p18(F71 N) vector prepared according to Example 1. The transformed bacteria were grown in YT culture medium. When the optical density was 0.5 at an absorbance of 600 nm, 0.5 mM IPTG was added to the culture medium, and the transformed *E. coli* BL21(DE3) was further cultured at 18 °C for 16 hours. The cultured cells were sonicated in a 50 mM Tris-HCl buffer solution (pH 8.0) including 5% glycerol, 5 mM β-mercaptoethanol, 0.2% Triton X-100 and 0.2 M NaCl, and then centrifuged to obtain the supernatant (10,000 g). The supernatant was applied to a Ni²⁺-NTA superflow column (Qiagen) in equilibrium with the buffer solution, washed using a washing buffer solution (50 mM Tris-HCl, pH 8.0, 5% glycerol, 5mM β-mercaptoethanol, 0.2% Triton X-100 and 1 M NaCl) having a volume 5 times that of the column, and eluted using an elution buffer solution (50 mM Tris-HCl, pH 8.0, 5% glycerol, 5 mM β-mercaptoethanol, 0.2% Triton X-100 and 0.2 M NaCl). The fractions including the fusion proteins were collected, and salts contained in the fractions were removed using Amicon Ultra-15 Centrifugal Filter(Milipore), and then the fractions were concentrated. The concentration of the purified protein was quantified using bovine serum albumin (BSA) as a standard.

Expression and purification properties of the fusion protein including ubiquitin and the fusion protein not including ubiquitin (control) were determined The results are shown in Table 1 below. The fusion protein including ubiquitin according to the present embodiment showed a high expression amount, and excellent hydrophilicity, yield, concentration, and purity.

**Table 1**

| | Fusion protein not including ubiquitin (control) | Fusion protein including ubiquitin |
|---|---|---|
| Expression amount | 50 mg/ℓ | 300 mg/ℓ |
| Expression status | Inclusion body | soluble |
| Purification yield | 20 mg/ℓ | 200 mg/ℓ |
| Concentration | 1.2 mg/Mℓ | 14 mg/Mℓ |
| Purity | 80% | 95% |

### Example 3 : Characterization of the cell-penetrating property and stability of the transmembrane fusion protein.

The cell-penetrating property of the transmembrane fusion protein prepared in Example 2 was measured in an animal model.

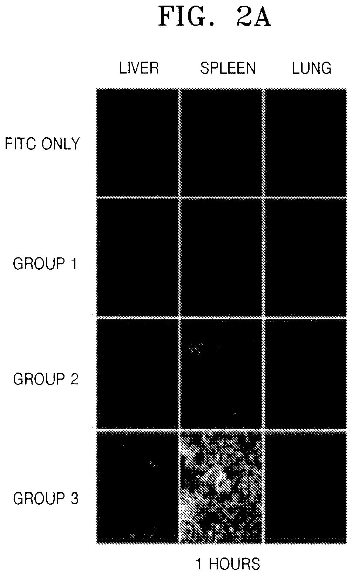

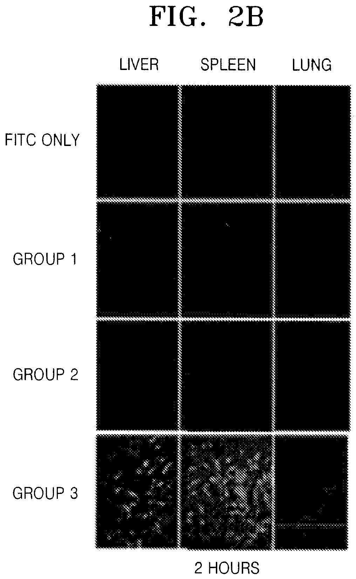

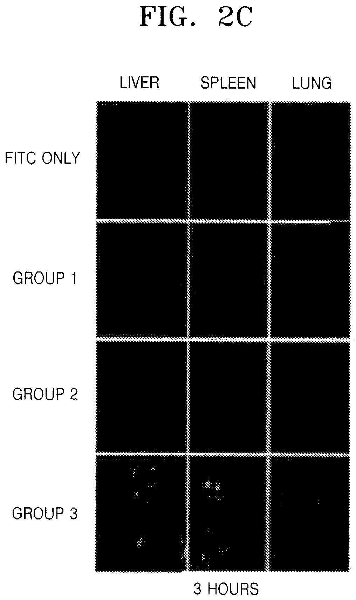

Seven week old nude mice (Balb/c mouse, Orient Bio, Inc., Korea) that were immunodeficient due to mutation in the major histocompatibility complex (MHC) were used as the animal model. Three groups of 3 mice were intraperitoneally injected with six hundred micrograms of a fusion protein at 1 *µg*/*µℓ.* The fusion proteins used for injection to a group of mice were the fusion protein including ubiquitin (Ub-NLS-kFGF4-p53-p18(F71 N) (Group 3), the fusion protein not including ubiquitin (NLS-kFGF4-p53-p18(F71 N) (Group 2), and a fusion protein including only p53-p18 (p53-p18(F71 N), (Group 1). As an additional control, a fourth group of 3 mice were intraperitoneally injected with 600 *µℓ* of a Tris buffer solution including FITC (FITC). Then, cells were obtained from the liver, spleen, and lung of one of the 3 mice at 1, 2 and 3 hours, respectively, after the injection. After washing the cells twice, the cells were stained at 4 °C for 1 hour with a mouse monoclonal anti-p18 antibody (Thermo Scientific Pierce, U.S.A.) diluted to a 1:100 ratio (v/v) with a phosphate buffer solution (PBS) including 0.1% bovine serum albumin and 0.05% sodium azide. Then, the cells were washed twice, and incubated with an FITC-binding secondary goat anti-mouse IgG-HRP (Santa Cruz Biotechnology, U.S.A.) diluted in PBS including 0.1 % bovine serum albumin at 4 °C for 30 minutes. The cells were washed twice, and the in vivo distribution was visualized using a fluorescence microscope (Nikon, Japan). Representative images are shown in Fig. 2A to Fig. 2C.

As shown in FIG. 2A to Fig. 2C, the amount of the fusion protein including ubiquitin present in spleen cells was larger than the amount of the fusion protein not including ubiquitin under the same condition. This indicates that the fusion protein including ubiquitin was transferred into the cell more efficiently. Thus it was determined that the transmembrane fusion protein including ubiquitin, after transfer into the cell, stably exists within the cell for a long period of time.

### Example 4 : Determination of the therapeutic effect of the transmembrane fusion protein in a cancer cell animal model

The therapeutic effect of p53-p18 protein administered in vivo in the form of the transmembrane fusion protein prepared as in Example 2 was determined using a xenograft mouse model transplanted with a colon cancer cell line (Wild-type p53-positive HCT116), Korean Cell Line Bank (KCLB).

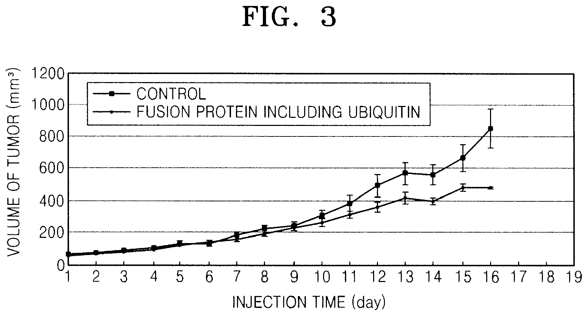

Fifty microliters of colon cancer cells HCT116 (1×10⁷) were subcutaneously injected into 6 week old female BALB/c nude mice. After 4 to 5 weeks, tumors were found in the mice. When the size of a tumor (width²×length/2) measured using a vernier caliper was 80 mm ³, 200 *µ*g of the control fusion protein (not including ubiquitin) or the fusion protein including ubiquitin were intravenously injected into 10 mice at 1 *µg*/*µℓ* daily for 16 days. Tumor size was monitored daily over the 16 day period. Fig. 3 shows a graph of tumor volume as a function of time after injections were begun.

Referring to FIG. 3, the injected fusion protein including ubiquitin inhibited tumor growth relative to the size of tumors observed in animals injected with the control fusion protein. By day 16, average tumor size in the group injected with the ubiquitin containing fusion protein was about 46% smaller than that observed in the group injected with the control fusion protein.

### Example 5. Expression and purification of additional transmembrane fusion proteins with p53-p18 as the biologically active molecule

Other transmembrane fusion protein expression vectors are prepared in a similar manner as described above in Example 1 by changing the order of the modules in the sequence of the transmembrane fusion protein, for example, locating the NLS between kFGF4 and p53, replacing the RGG at the C-terminus of the ubiquitin with RGA, or replacing each of the seven Lys in ubiquitin with Arg.

Additional transmembrane fusion protein expression vectors analogous to pET-Ub-NLS-kFGF4-p53-p18(F71 N) for a fusion protein with p53-p18 as the biologically active molecule are prepared replacing the kFGF4 MTS in the encoded fusion protein with another MTS. A transmembrane fusion protein expression vector for a fusion protein with p53-p18 as the biologically active molecule is made encoding an MTS selected from SEQ ID NOS: 2-15.

Each of these transmembrane fusion proteins is expressed and isolated as described in Example 2.

### Example 6. Expression and purification of transmembrane fusion proteins including alternate biologically active molecules.

Additional transmembrane fusion protein expression vectors are made as described in Example 1, each including the coding sequence of an alternate biologically active molecule instead of p53-p18. For each of these recombinant vectors prepared, a polynucleotide fragment of the known cDNA sequence of the biolgically active molecule is synthesized by PCR for subsequent cloning into the expression vector as described above and is cloned into the expression vector using methods known in the art.

A transmembrane fusion protein expression vector is made for each of the following biologically active molecules: neuroregulin 1, PTEN tumor suppressor, ARF tumor suppressor, APC, CD95, folliculin, MEN1, BRCA1, Von Hippel-Lindau tumor suppressor, RKIP, nm23, endostatin, insulin, insulin-like growth factor 1 (IGF-1), growth factor, erythropoietin, granulocyte-colony stimulating factor (G-CSF), granulocyte/macrophage-colony stimulating factor (GM-CSF), interferon-α, interferon-β, interferon-γ, interleukin-1 α and β, interleukin-3, interleukin-4, interleukin-6, interleukin-2, epidermal growth factor (EGF), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRH-II), gonadorelin, goserelin, histrelin, leuprorelin, lypressin, octreotide, oxytocin, pitressin, secretin, sincalide, terlipressin, thymopentin, thymosine α1, triptorelin, bivalirudin, carbetocin, cyclo-sporine, exedine, lanreotide, luteinizing hormone-releasing hormone (LHRH), nafarelin, parathyroid hormone, pramlintide, enfuvirtide (T-20), thymalfasin, and ziconotide.

Each of these transmembrane fusion proteins is expressed and isolated as described in Example 2.

The terminology used herein is for the purpose of describing particular embodiments only and is not intended to be limiting of the invention. The terms "a" and "an" do not denote a limitation of quantity, but rather denote the presence of at least one of the referenced item. The terms "comprising", "having", "including", and "containing" are to be construed as open-ended terms (i.e. meaning "including, but not limited to"). The modifier "about" used in connection with a quantity is inclusive of the stated value and has the meaning dictated by the context (e.g., includes the degree of error associated with measurement of the particular quantity),

Recitation of ranges of values are merely intended to serve as a shorthand method of referring individually to each separate value falling within the range, unless otherwise indicated herein, and each separate value is incorporated into the specification as if it were individually recited herein. The endpoints of all ranges are included within the range and independently combinable.

All methods described herein can be performed in a suitable order unless otherwise indicated herein or otherwise clearly contradicted by context. No language in the specification should be construed as indicating any non-claimed element as essential to the practice of the invention as used herein.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

It should be understood that the exemplary embodiments described herein should be considered in a descriptive sense only and not for purposes of limitation. Descriptions of features or aspects within each embodiment should typically be considered as available for other similar features or aspects in other embodiments.

## Claims

1. A transmembrane fusion protein having the capability of delivering a biologically active molecule into a cell or a nucleus,comprising:
ubiquitin or a ubiquitin-like protein;
a membrane translocation sequence linked to the C-terminus of the ubiquitin or ubiquitin-like protein; and
the biologically active molecule linked to the C-terminus of the membrane translocation sequence.

2. The transmembrane fusion protein of claim 1, wherein the ubiquitin or ubiquitin-like protein is a mutant ubiquitin that has Arg replacing Lys in an amino acid sequence of wild-type ubiquitin or that has the RGG residues of the C-terminus of wild-type ubiquitin replaced with RGA residues.

3. The transmembrane fusion protein of any one of claim 1 to 2, wherein the ubiquitin or ubiquitin-like protein has a protease recognition amino acid sequence that is cleaved by a protease.

4. The transmembrane fusion protein of any one of claim 1 to 3, wherein the ubiquitin or ubiquitin-like protein is a ubiquitin-like protein selected from the group consisting of Nedd8, SUMO-1, SUMO-2, NUB1, PIC1, UBL3, UBL5, and ISG15.

5. The transmembrane fusion protein of any one of claim 1 to 4, wherein the membrane translocation sequence comprises a polypeptide of a sequence selected from the group consisting of SEQ ID NOS: 1 to 15.

6. The transmembrane fusion protein of any one of claim 1 to 5, wherein the biologically active molecule is selected from the group consisting of p53, p18 (CDKN2C), neuroregulin 1, PTEN tumor suppressor, ARF tumor suppressor, APC, CD95, folliculin, MEN1, BRCA1, Von Hippel-Lindau tumor suppressor, RKIP, nm23, endostatin, insulin, insulin-like growth factor 1 (IGF-1), growth factor, erythropoietin, granulocyte-colony stimulating factor (G-CSF), granulocyte/macrophage-colony stimulating factor (GM-CSF), interferon-α, interferon-β, interferon-γ, interleukin-1 α and β, interleukin-3, interleukin-4, interleukin-6, interleukin-2, epidermal growth factor (EGF), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRH-II), gonadorelin, goserelin, histrelin, leuprorelin, lypressin, octreotide, oxytocin, pitressin, secretin, sincalide, terlipressin, thymopentin, thymosine α1, triptorelin, bivalirudin, carbetocin, cyclo-sporine, exedine, lanreotide, luteinizing hormone-releasing hormone (LHRH), nafarelin, parathyroid hormone, pramlintide, enfuvirtide (T-20), thymalfasin, and ziconotide.

7. The transmembrane fusion protein of any one of claim 1 to 6, further comprising a nuclear localization signal domain.

8. The transmembrane fusion protein of claim 7, wherein the nuclear localization signal domain comprises a polypeptide having SEQ ID NO: 26.

9. A polynucleotide encoding the transmembrane fusion protein of any one of claim 1 to 8.

10. A recombinant vector comprising: the polynucleotide of claim 9; and a promoter operatively linked to the polynucleotide.

11. A host cell including the polynucleotide of claim 9, or including the recombinant vector of claim 10, wherein the host cell is preferably transformed by the polynucleotide of claim 9 or by the recombinant vector of claim 10.

12. An in vitro method of delivering a transmembrane fusion protein to a cell, the method comprising
contacting the transmembrane fusion protein of any one of claim 1 to 8 with the cell.

13. The transmembrane fusion protein according to any one of claims 1 to 8 for use as a medicament.

14. A method of producing a transmembrane fusion protein, comprising.
culturing the host cell of claim 13 under conditions which allow expression of the transmembrane fusion protein; and
recovering the transmembrane fusion protein from the host cell culture.

## Patentansprüche

1. Transmembranfusionsprotein mit der Fähigkeit, ein biologisch aktives Molekül in eine Zelle oder einen Kern abzugeben, umfassend:
Ubiquitin oder ein Ubiquitin-ähnliches Protein;
eine Membrantranslokationssequenz, verknüpft mit dem C-Terminus des Ubiquitins oder Ubiquitin-ähnlichen Proteins, und
das biologisch aktive Molekül, verknüpft mit dem C-Terminus der Membrantranslokationssequenz.

2. Transmembranfusionsprotein nach Anspruch 1, wobei es sich bei dem Ubiquitin oder Ubiquitin-ähnlichen Protein um ein mutantes Ubiquitin handelt, bei dem Arg in einer Aminosäuresequenz von Wildtyp-Ubiquitin Lys ersetzt oder bei dem die RGG-Reste des C-Terminus von Wildtyp-Ubiquitin durch RGA-Reste ersetzt sind.

3. Transmembranfusionsprotein nach einem der Ansprüche 1 bis 2, wobei das Ubiquitin oder Ubiquitin-ähnliche Protein eine Proteaseerkennungs-Aminosäuresequenz aufweist, die durch eine Protease gespalten wird.

4. Transmembranfusionsprotein nach einem der Ansprüche 1 bis 3, wobei es sich bei dem Ubiquitin oder Ubiquitin-ähnlichen Protein um ein Ubiquitin-ähnliches Protein handelt, ausgewählt aus der Gruppe, bestehend aus Nedd8, SUMO-1, SUMO-2, NUB1, PIC1, UBL3, UBL5 und ISG15.

5. Transmembranfusionsprotein nach einem der Ansprüche 1 bis 4, wobei die Membrantranslokationssequenz ein Polypeptid mit einer aus der Gruppe, bestehend aus SEQ ID NO: 1 bis 15, ausgewählten Sequenz umfasst.

6. Transmembranfusionsprotein nach einem der Ansprüche 1 bis 5, wobei das biologisch aktive Molekül aus der Gruppe ausgewählt ist, bestehend aus p53, p18 (CDKN2C), Neuroregulin 1, PTEN Tumorsuppressor, ARF Tumorsuppressor, APC, CD95, Folliculin, MEN1, BRCA1, Von Hippel-Lindau Tumorsuppressor, RKIP, nm23, Endostatin, Insulin, Insulin-ähnlichem Wachstumsfaktor 1 (IGF-1), Wachstumsfaktor, Erythropoietin, Granulozyten-Koloniestimulierendem Faktor (G-CSF), Granulozyten/Makrophagen-Koloniestimulierendem Faktor (GM-CSF), Interferon-α, Interferon-β, Interferon-γ, Interleukin-1α und β, Interleukin-3, Interleukin-4, Interleukin-6, Interleukin-2, Epidermalem Wachstumsfaktor (EGF), Calcitonin, Adrenocorticotropem Hormon (ACTH), Tumornekrosefaktor (TNF), Atobisban, Buserelin, Cetrorelix, Deslorelin, Desmopressin, Dynorphin A (1-13), Elcatonin, Eleidosin, Eptifibatid, Wachstumshormon Releasing Hormon-II (GHRH-II), Gonadorelin, Goserelin, Histrelin, Leuprorelin, Lypressin, Octreotid, Oxytocin, Pitressin, Sekretin, Sincalid, Terlipressin, Thymopentin, Thymosin α1, Triptorelin, Bivalirudin, Carbetocin, Cyclosporin, Exedin, Lanreotid, Luteinisierendes Hormon-Releasing Hormon (LHRH), Nafarelin, Parathormon, Pramlintid, Enfuvirtid (T-20), Thymalfasin und Ziconotid.

7. Transmembranfusionsprotein nach einem der Ansprüche 1 bis 6, ferner umfassend eine Kernlokalisationssignal-Domäne.

8. Transmembranfusionsprotein nach Anspruch 7, wobei die Kernlokalisationssignal-Domäne ein Polypeptid mit SEQ ID NO: 26 umfasst.

9. Polynucleotid, welches das Transmembranfusionsprotein nach einem der Ansprüche 1 bis 8 kodiert.

10. Rekombinanter Vektor, umfassend: das Polynucleotid nach Anspruch 9 und einen mit dem Polynucleotid funktionell verknüpften Promoter.

11. Wirtszelle, welche das Polynucleotid nach Anspruch 9 einschließt oder den rekombinanten Vektor nach Anspruch 10 einschließt, wobei die Wirtszelle vorzugsweise mit dem Polynucleotid nach Anspruch 9 oder mit dem rekombinanten Vektor nach Anspruch 10 transformiert ist.

12. *In*-*vitro*-Verfahren zum Abgeben eines Transmembranfusionsproteins an eine Zelle, wobei das Verfahren umfasst
In-Kontakt-Bringen des Transmembranfusionsproteins nach einem der Ansprüche 1 bis 8 mit der Zelle.

13. Transmembranfusionsprotein gemäß einem der Ansprüche 1 bis 8 zur Verwendung als Arzneimittel.

14. Verfahren zum Herstellen eines Transmembranfusionsproteins, umfassend
Züchten der Wirtszelle nach Anspruch 13 unter Bedingungen, die eine Expression des Transmembranfusionsproteins ermöglichen, und
Gewinnen des Transmembranfusionsproteins aus der Wirtszellkultur.

## Revendications

1. Protéine de fusion transmembranaire ayant la capacité d'amener une molécule active biologiquement dans une cellule ou un noyau, comprenant :
de l'ubiquitine ou une protéine de type ubiquitine ;
une séquence de translocation membranaire liée à l'extrémité C-terminale de l'ubiquitine ou de la protéine de type uniquitine ; et
la molécule active biologiquement liée à l'extrémité C-terminale de la séquence de translocation membranaire.

2. Protéine de fusion transmembranaire selon la revendication 1, dans laquelle l'ubiquitine ou la protéine de type ubiquitine est une ubiquitine mutante dans laquelle Arg remplace Lys dans une séquence d'acides aminés de l'ubiquitine de type sauvage ou dans laquelle les résidus RGG de l'extrémité C-terminale de l'ubiquitine de type sauvage sont remplacés par des résidus RGA.

3. Protéine de fusion transmembranaire selon la revendication 1 ou 2, dans laquelle l'ubiquitine ou la protéine de type ubiquitine possède une séquence d'acides aminés de reconnaissance de protéase qui est clivée par une protéase.

4. Protéine de fusion transmembranaire selon l'une quelconque des revendications 1 à 3, dans laquelle l'ubiquitine ou la protéine de type ubiquitine est une protéine de type ubiquitine choisie dans le groupe constitué de Nedd8, SUMO-1, SUMO-2, NUB1, PIC1, UBL3, UBL5 et ISG15.

5. Protéine de fusion transmembranaire selon l'une quelconque des revendications 1 à 4, dans laquelle la séquence de translocation membranaire comprend un polypeptide selon une séquence choisie dans le groupe constitué des SEQ ID NO : 1 à 15.

6. Protéine de fusion transmembranaire selon l'une quelconque des revendications 1 à 5, dans laquelle la molécule active biologiquement est choisie dans le groupe constitué de p53, p18 (CDKN2C), neuroréguline 1, suppresseur de tumeur PTEN, suppresseur de tumeur ARF, APC, CD95, folliculine, MEN1, BRCA1, suppresseur de tumeur Von Hippel-Lindau, RKIP, nm23, endostatine, insuline, facteur de croissance 1 analogue à l'insuline (IGF-1), facteur de croissance, érythropoiétine, facteur de stimulation des colonies de granulocytes (G-CSF), facteur de stimulation des colonies de granulocyres/macrophages (GM-CSF), interféron-α, interféron-β, interféron-γ, interleukine-1 α et β, interleukine-3, interleukine-4, interleukine-4, interleukine-6, interleukine-2, facteur de croissance épidermique (EGF), calcitonine, hormone adrénocorticotropique (ACTH), facteur de nécrose tumorale (TNF), atobisban, buséréline, cétrorélix, desloréline, desmopressine, dynorphine A (1-13), elcatonine, éléidosine, eptifibatide, hormone de libération de l'hormone de croissance II (GHRH-II), gonadoréline, goséréline, histréline, leuproréline, lypressine, octréotide, oxytocine, pitressine, secrétine, sincalide, terlipressine, thymopentine, thymosine α1, triptoréline, bivalirudine, carbétocine, cyclosporine, exédine, lanréotide, hormone de libération de l'hormone de lutéinisation (LHRH), nafaréline, parathormone, pramlintide, enfuvirtide (T-20), thymalfasine et ziconotide.

7. Protéine de fusion transmembranaire selon l'une quelconque des revendications 1 à 6, comprenant en outre un domaine de signalisation de localisation nucléaire.

8. Protéine de fusion transmembranaire selon la revendication 7, dans laquelle le domaine de signalisation de localisation nucléaire comprend un polypeptide ayant la SEQ ID NO : 26.

9. Polynucléotide codant pour la protéine de fusion transmembranaire selon l'une quelconque des revendications 1 à 8.

10. Vecteur recombiné comprenant : le polynucléotide selon la revendication 9 ; et un promoteur lié fonctionnement au polynucléotide.

11. Cellule hôte comprenant le polynucléotide selon la revendication 9, ou comprenant le vecteur recombiné selon la revendication 10, la cellule hôte étant de préférence transformée par le polynucléotide selon la revendication 9 ou par le vecteur recombiné selon la revendication 10.

12. Procédé *in vitro* pour administrer une protéine de fusion transmembranaire à une cellule, le procédé comprenant
la mise en contact de la protéine de fusion transmembranaire selon l'une quelconque des revendications 1 à 8 avec la cellule.

13. Protéine de fusion transmembranaire selon l'une quelconque des revendications 1 à 8 destinée à être utilisée comme médicament.

14. Procédé de production d'une protéine de fusion transmembranaire, comprenant.
la culture de la cellule hôte selon la revendication 13 dans des conditions qui permettent l'expression de la protéine de fusion transmembranaire ; et
la récupération de la protéine de fusion transmembranaire à partir de la culture de la cellule hôte.
